# EUROPEAN PATENT APPLICATION

(11) **EP 1 541 670 A1**
(43) Date of publication of application: **15.06.2005**
(21) Application number: 03792841.3
(22) Date of filing: 26.08.2003
(51) Int. Cl.: C12M 3/00, C12M 1/12, C12M 1/34

(54) **MICROCHAMBER FOR NERVE CELL CULTURE**

(30) Priority: 26.08.2002 JP 2002245903
(71) Applicant: Japan Science and Technology Agency, Kawaguchi-shi, Saitama 332-0012 (JP)
(72) Inventor: YASUDA, Kenji, Koto-ku, Tokyo 135-0052 (JP)
(74) Representative: Calamita, Roberto
(86) International application number: PCT/JP2003/010759
(87) International publication number: WO 2004/018617

(57) **Abstract**

A microchamber for culturing nerve cells which comprises cell-sized electrode arrays located on a transparent glass substrate, microchamber arrays of 10 µm or more in thickness for aligning cells provided thereon, and a semipermeable membrane, which has such a pore size that the cells cannot pass therethrough and is optically transparent to focused beam, provided on the microchamber to coat it thereby blocking the leakage of the cells from the chamber. This microchamber is further provided with a unit of allowing the replacement of a solution in the solution replacing unit, wherein a culture liquor is circulated, on the upper face of the semipermeable membrane; a unit of continuously and optically monitoring changes in the conditions of the cells in the microchamber arrays; and a unit of continuously measuring potential changes in each nerve cell and a unit for combining the both units. To clarify the learning process of cells, changed in stimulus responses are measured over a long time while completely controlling the network system and preventing the invasion with bacteria, etc.

## Description

### Technical Field

The invention according to the present application relates to a novel microchamber for nerve cell culture capable of culturing nerve cells one by one while observing the state of the cells under a microscope and at the same time, measuring the potential change of the cells.

### Background Art

Recent advances in neuroscience are remarkable and a variety of methods utilizing light, magnetic fields and chemical substances have been developed and used for researches in order to understand the cerebral function. Although it is the common practice to clarify the cerebral function in vivo particularly with regards to its high-level information processing capacity, maintenance of a stable sample state and reproduction of sample conditions cannot be attained completely because of complex neural networks. Many studies have been carried out to artificially construct a relatively simple neural network from a small number of nerve cells and clarify the information processing function of a cell network under a completely controlled environment. Examples of such studies include Dichter, M.A., Brain Res., 149, 279-293(1978), Mains R.E., Patterson P.H., J. Cell. Biol., 59, 329-345(1973), Potter S.M., DeMarse T.B., J. Neurosci. Methods, 110, 17-24(2001) and Jimbo Y., Tateno T., Robinson H.P.C., Biophys. J., 76, 670-678(1999).

For the analysis of an information processing model having each of nerve cells as the minimum unit, a multipoint simultaneous measurement technology and a controlling technology of a cell network pattern are important. The measurement technology of an action potential of a nerve cell had, at the initial stage thereof, problems that measurement points at the same time were three at most and cells died several hours after beginning of the measurement, because a method, such as patch clamping, mainly adopted for it gave damage to the cells. Owing to the recent development of a culture assay method of nerve cells on an electrode array (MEAS) substrate, the above-described problems are overcome and cultivation even for a period as long as several weeks can be carried out now.

Many studies have conventionally been made on the technology of controlling the network pattern of nerve cells based on the chemical or physical method. As one example of the chemical method, Letourneau, et al. have succeeded in drawing of a pattern with a cell-adhesive substratum such as laminin on the surface of a substrate on which nerve cells are to be cultivated and causing neurite outgrowth along the pattern. This is reported, for example, in Letourneau P.C., Dev. Biol., 66, 183-196(1975). On the other hand, the physical method reported is to cultivate nerve cells on a substrate having a surface on which steps serving as a barrier against the extension of nerve cells have been constructed. According to this report, when the barrier has a height of 10 µm or greater, the extension or movement of nerve cells can be limited (Stopak D. et al., Dev. Biol., 90, 383-398(1982), or Hirono T., Torimitsu K., Kawana A., Fukuda J., Brain Res., 446, 189-194(1988), etc.).

The electrode array substrate technology invented by the above-described background art has however difficulty in complete control of the spatial arrangement of cells because it has no steric hindrance on the substrate. In addition, it is difficult to prevent the invasion of bacteria from the outside world such as a circulating culture solution when the spatial arrangement of cells is controlled by the steric structure according to the background art.

An object of the invention according to the present application is, in order to overcome the above-described problems of the background art and clarify the learning procedure of cells, to provide a novel technological system capable of measuring a change in stimulus response of a neural network for a long period of time without invasion of bacteria while completely controlling the network pattern.

### Disclosure of the Invention

In a first aspect of the invention according to the present application, there is thus provided, as a solution to the above-described problems, a microchamber for nerve cell culture, which comprises a plurality of electrode patterns for measuring a potential change of nerve cells, a plurality of compartment walls thereover for confining the neural cells in a specific spatial arrangement, and an optically transparent semipermeable membrane laid over the compartment walls. More specifically, the microchamber for nerve cell culture according to the present invention has, on a transparent glass substrate, cell-sized electrode arrays, microchamber arrays of at least 10 µm thick for aligning cells, and a semipermeable membrane which covers the upper surface of the microchamber so as to block the cells from coming out of the chamber, has a pore size small enough to disturb the passage of cells through the membrane, and is optically transparent to convergent light.

In a second aspect, a third aspect, a fourth aspect and a fifth aspect of the invention, there are also provided the microchambers for nerve cell culture according to the first aspect of the invention, wherein the electrode patterns are optically transparent electrodes; the electrode patterns are at least three electrodes for permitting independent measurement; the number of cell culture regions separated by the plurality of compartment walls is 3 or greater; and each of the electrodes corresponds to each of the regions, respectively.

The microchamber for nerve cell culture according to the present invention is further provided with a unit permitting the replacement of a solution in the solution replacement section, through which a culture solution is circulated, on the upper surface of the semipermeable membrane. It is still further provided with a unit of continuously and optically monitoring changes in the conditions of the cells in the microchamber array, a unit of continuously measuring a potential change of each nerve cell and a unit for combining the both units.

### Brief Description of Drawings

FIG 1 is a schematic view illustrating a basic constitution of a multielectrode array long-term cultivation microscopic observation system according to the present application;
FIG 2 is a micrograph of a multielectrode array and a microchamber;
FIG 3 illustrates a basic concept of adhesion of a semipermeable membrane to a substrate;
FIG 4 is a micrograph of the external appearance of a multielectrode array chip;
FIG 5 is a schematic view of a multielectrode assay unit and a photograph of the external appearance of this unit;
FIG 6 is a photograph of the external appearance of a long-term cultivation microscopic system used for measurement and observation; and
FIG 7 is a micrograph showing rat cerebeller granular cells on the multielectrode array chip.

### Best Mode for Carrying out the Invention

The invention according to the present application has characteristics as described above. The embodiment of the invention will next be described.

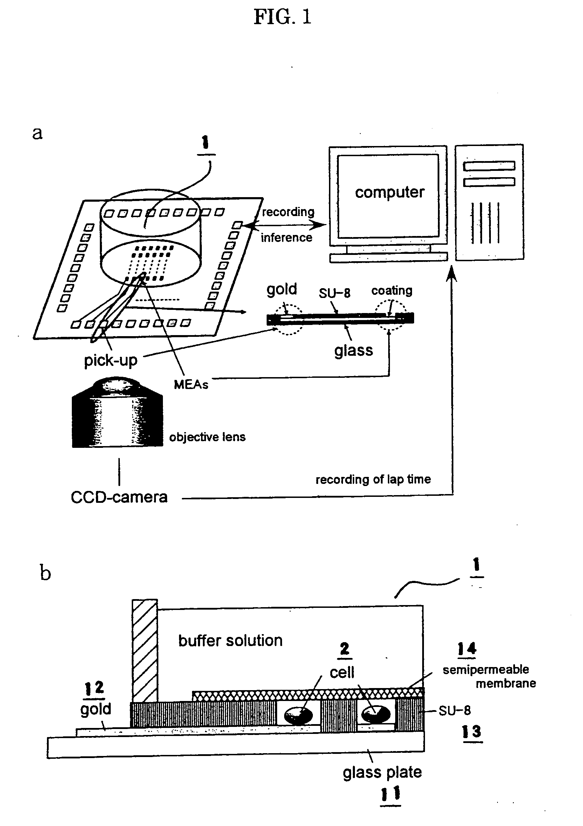

For example, accompanying drawing FIG 1 illustrates one example of a multielectrode array long-term cultivation microscopic observation system using the microchamber for nerve cells according to the present invention. As illustrated in FIG 1(a), a multielectrode array chip (1) which is the heart of this system is installed in the microscope observation system, whereby measurement of lap time from an optical system to a controlling computer via a CCD camera, recording of electrical signals from the electrode array, and electrical stimulation to the cells from each electrode array terminal can be carried out. This enables simultaneous measurement and recording of electric signals and optical data.

FIG 1(b) illustrates a partial cross-sectional view of one example of the multielectrode array chip (1) which is the heart of this system. On a slide glass (11) as thin as 0.18 mm which permits observation using a 100X objective lens, an electrode (12) array layer is laid. The electrode surface is covered with a photocurable resin "SU-8" (product of Micro Chem Inc., USP 4882245, a thick photoresist material which is an epoxy polymer and is polymerized at a portion exposed to light) having a viscosity adjusted to give a thickness of 25 µm and at the same time, this resin layer is partially removed by etching, whereby compartment walls (13) defining holes (microchamber arrays) which have the cells (2) confined therein are formed. It is needless to say that as well as the above-described SU-8, any resin is usable insofar as it is a relatively thick resist material which is photocurable.

Laminin or collagen is applied to the surface of the gold electrode (12) on which the cells (2) are placed. The upper surface of the microchamber arrays having the cells (2) confined therein is covered with a semipermeable membrane (14) in order to prevent contamination of the cells (2) from the outside and at the same time, to prevent the escape of the cells (2) to the outside. The culture solution buffer on the chip is constantly circulated to keep the solution fresh. In this embodiment, a gold electrode is used, but an optically transparent electrode such as ITO can be used instead.

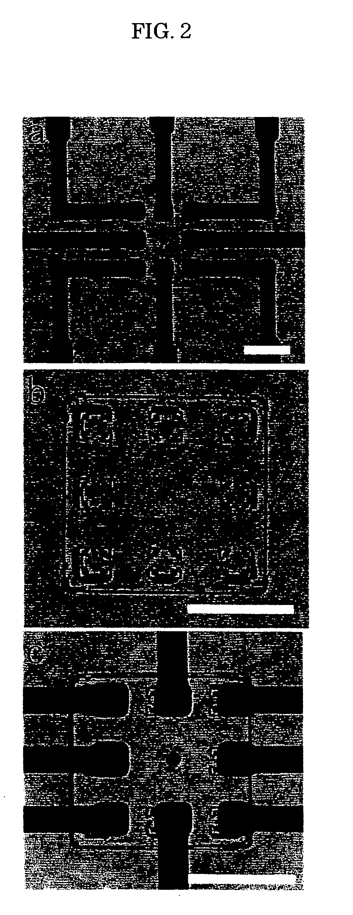

The multielectrode array chip will next be described more specifically. In FIG 2, microscopic photographs of the substrate in each processing stage are shown. The measure bar shown in the lower right hand corner of each photograph shows the length of 100 µm. FIG 2(a) is an electrode pattern formed by evaporating Cr of 100Å and Au of 1000Å over a slide glass substrate in that order, followed by application of a photoresist chemical, exposure to light, development and etching. The electrode has a size of 30 µm on a side, in consideration of the size of the microchamber to be used in combination. FIG 2(b) is a microchamber array formed by applying the above-described photocurable resin "SU-8" to the slide glass substrate and drying, followed by exposure and etching to form a desired pattern. In this example, eight walls of 30 µm on a side are placed in consideration of the positions of the electrodes in FIG 2(a). The height of the wall made by "SU-8" is 25 µm (as measured by a step height scale) and the width of the wall of the microchamber array is about 1 to 5 µm as can be seen from the drawing. Use of "SU-8" in such a manner enables to form a structure having a high height/width ratio. FIG 2(c) is an actual combined example of the electrode of FIG 2(a) and the microstructure of FIG 2(b). For the cultivation of nerve cells in the example described later, a multielectrode array chip in such a form is employed.

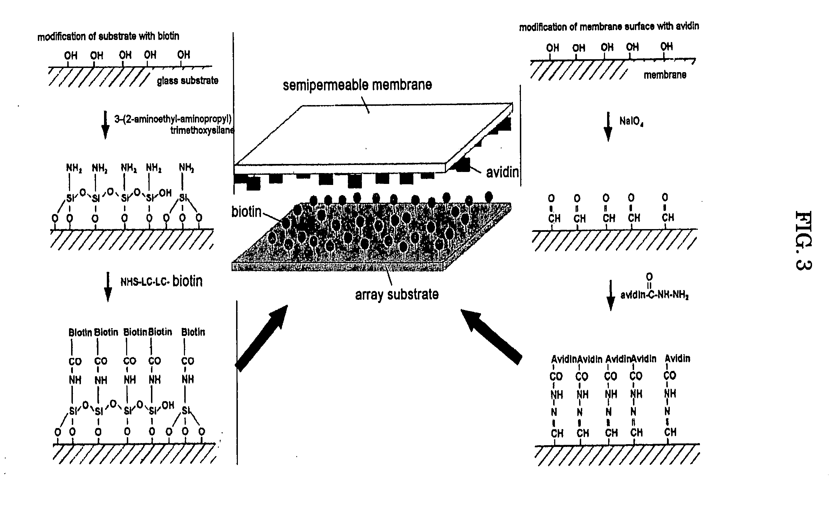

In the next place, one of the fixing methods of the semipermeable membrane which is a lid covering therewith the upper surface of the multielectrode array chip will be described briefly. In FIG 3, the procedures of a method of modifying the surface of the substrate (11) with biotin and a method of modifying the semipermeable membrane with avidin are briefly shown. First, an amino group is added to the surface of the substrate (11), followed by the reaction with biotin having an epoxy group introduced at the terminal thereof. The semipermeable membrane (14) is composed, for example, of cellulose so that cleavage of a portion of the cyclopentose ring of this polysaccharide is caused, followed by the reaction with the amino group added to the terminal of avidin, whereby an avidin-modified semipermeable membrane is prepared. The semipermeable membrane (14) is fixed to the substrate (11) by adhesion through the avidin-biotin bond.

When the above-described photocurable resin SU-8 is used, it can be fixed to the substrate in the following manner because SU-8 has a reactive epoxy group. The surface of the substrate (11) baked prior to exposure to light is caused to react with the amino group of a protein, followed by exposure to light; or after formation of a pattern using SU-8, the surface of the pattern is coated with SiO₂ by sputtering, followed by the addition of the epoxy group onto the coated surface by silane coupling, whereby covalent bonding with the amino group of a protein is caused.

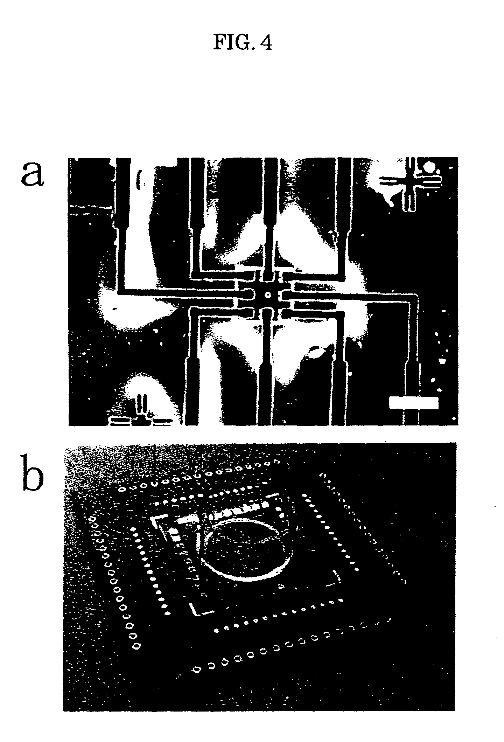

FIG 4(a) is a micrograph of the whole image of a multielectrode array chip thus manufactured. The length of the measure bar is 150 µm. FIG 4(b) is a photograph of a slide grass substrate of this multielectrode array chip fixed to a holder. This slide glass has a size of 3 cm × 3 cm. Upon actual cultivation and observation, the multielectrode array chip fixed to the holder as illustrated in FIG 4(b) is placed for measurement on a multielectrode primary amplifier attached to a microscope stage.

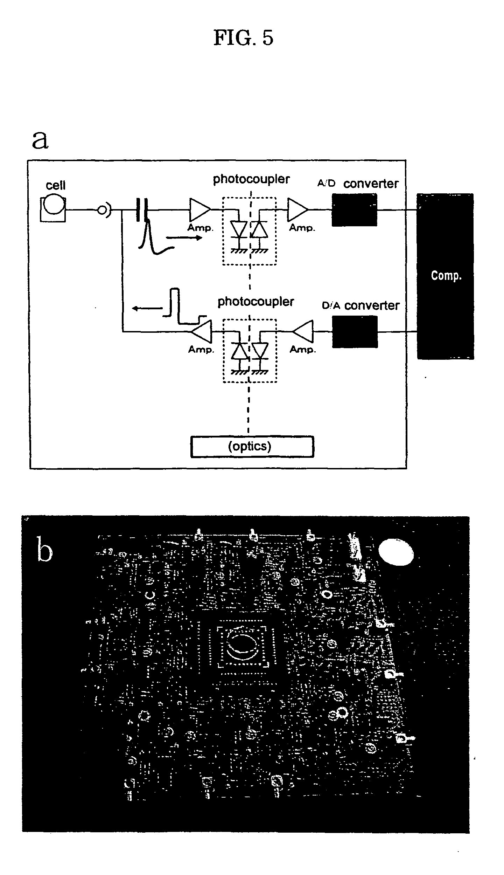

The actual measurement and cultivation system having a multielectrode array chip placed thereon will next be described briefly. FIG. 5 is a schematic view of a system which gives stimulus to nerve cells and electrically measures ignition of cells in practice. This apparatus is characterized in that stimulation of cells and measurement can be carried out by one electrode placed in the multielectrode array; continuous observation of the cell network form can be carried out optically; and the cells and an information recording apparatus are insulated at a ground level in the primary amplifier by optical connection. Even during long term cultivation, grounding prevents the spike signals from reaching the cells. FIG 5(a) is a schematic view of this system, while FIG. 5(b) is a photograph of the primary amplifier section of this apparatus. Nothing exists at the center of the primary amplifier substrate as illustrated in FIG. 5(b), which enables microscopic observation. The substrate is placed directly on the stage of the microscope. As a mounting substrate, a multilayer substrate having four layers is used, in which the top and bottom layers are ground planes. As described above, these ground planes are, at the inside and outside thereof, isolated each other. The ground plane on the cell side is driven by a battery, while that on the controlling computer is driven by a power supply. This system is a 12-channel type test model, but is able to have more channels by increasing the mounting density.

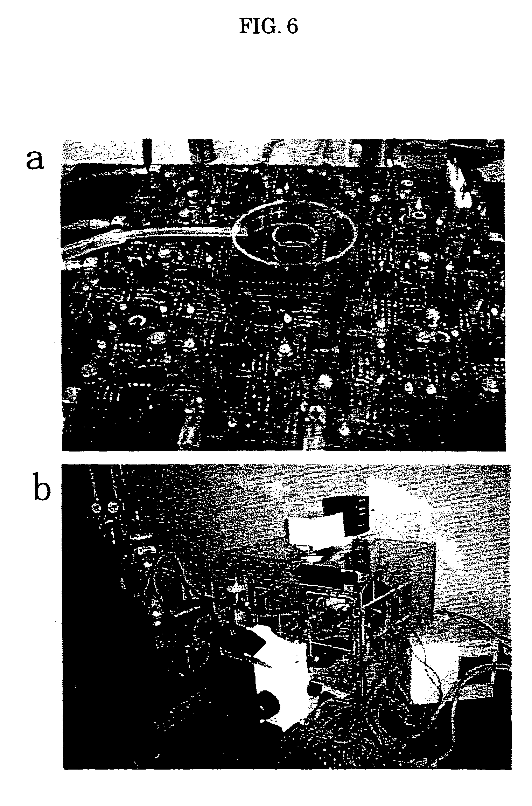

FIG 6 illustrates a microscopic system on which a multielectrode array chip is placed for cultivation. FIG 6(a) is a photograph of the primary amplifier substrate fixed onto the stage of the microscopic system and a chip fixed onto the substrate. A 5% CO₂-containing air having saturated vapor pressure is heated to 37°C and sprayed directly to the chip on the substrate. No reflux system of a culture solution is installed to this system in the photograph, but upon cultivation, continuous cultivation is carried out while replacing the culture solution with a new one through two SUS capillaries. As illustrated in FIG 6(b), the whole microscopic system is wrapped by a thermostatic chamber to stabilize the temperature of the whole system including the microscope.

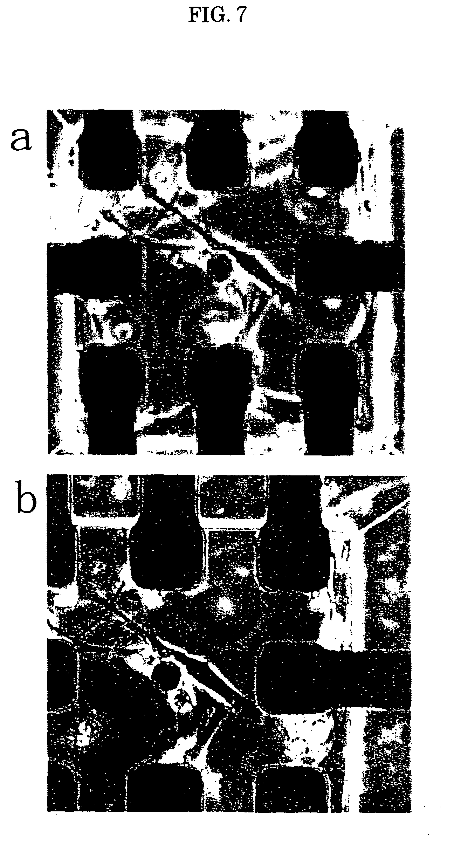

FIG 7 is a photograph of the rat cerebellar granular cells cultivated by this system, which was taken using a 40X objective lens. It was observed that the cells hermetically sealed in the microchamber array formed a network without escaping from the chamber. Mixture of impurities such as *Escherichia coli* from the environment was not observed at all. It has been understood from the observation that the structure of the microchamber achieved expected performances.

The present invention is not limited by the above-described examples. It is needless to say that the details of the structure can be modified in various ways.

### Industrial Applicability

As described above, the present invention makes it possible to continuously measure the morphological changes or changes in electrical properties of nerve cells by carrying out cultivation for a long time while controlling the network spatial arrangement of each cell.

## Claims

1. A microchamber for nerve cell culture, which comprises a plurality of electrode patterns on a substrate for measuring a potential change of nerve cells, a plurality of compartment walls over the patterns for confining the nerve cells in a specific spatial arrangement, and an optically transparent semipermeable membrane laid over the compartment walls.

2. The microchamber for nerve cell culture according to Claim 1, wherein the electrode patterns are optically transparent electrodes.

3. The microchamber for nerve cell culture according to Claim 1 or 2, wherein the electrode patterns are at least three electrodes capable of carrying out measurement independently.

4. The microchamber for nerve cell culture according to Claim 1, wherein regions of the cells separated by the plurality of compartment walls are at least three.

5. The microchamber for nerve cell culture according to any one of Claims 1 to 4, wherein with regards to the electrode patterns and the regions separated by the plurality of compartments, the electrodes correspond one-to-one with the regions.
